(19) Europäisches Patentamt

European Patent Office

Office européen des brevets

(11) **EP 0 814 071 B1**

(12) **EUROPEAN PATENT SPECIFICATION**

(45) Date of publication and mention
of the grant of the patent:
**02.05.2002 Bulletin 2002/18**

(51) Int Cl.[7]: **C07C 1/04**

(21) Application number: **97110120.9**

(22) Date of filing: **20.06.1997**

(54) **Process for preparing compounds**

Verfahren zur Herstellung von Verbindungen

Procédé de préparation de composés

(84) Designated Contracting States:
**DE FR GB**

(30) Priority: **21.06.1996 JP 16166496**

(43) Date of publication of application:
**29.12.1997 Bulletin 1997/52**

(73) Proprietor: **FURUKAWA DENCHI KABUSHIKI KAISHA**
**Yokohama-shi, Kanagawa-ken (JP)**

(72) Inventors:
 • **Sawa, Haruo, Furukawa Denchi K.K.,**
 **Iwakijigyosho**
 **Iwaki-shi, Fukushima (JP)**

 • **Abe, Hidetoshi, Furukawa Denchi K.K.**
 **Iwakijigyosho**
 **Iwaki-shi, Fukushima (JP)**

(74) Representative: **Modiano, Guido, Dr.-Ing. et al**
**Modiano, Josif, Pisanty & Staub,**
**Baaderstrasse 3**
**80469 München (DE)**

(56) References cited:
**FR-A- 2 322 115**

## Description

Field of the Invention

This invention relates to a novel process for preparing hydrocarbon compounds comprising ethane, propane, butane, pentane, hexane and heptane and to a novel process for preparing such hydrocarbon compounds additionally comprising methane, especially to a process capable of preparing, at around a normal temperature, such compounds which are difficult to produce by a conventional process or which can be produced only at a high temperature.

Prior Art

In conventional processes for preparing a desired compound, for example, by organic synthesis, raw materials are introduced into a reaction vessel in a simple manner, the processing conditions such as pressure, temperature, and the like are appropriately selected, and the synthesis is carried out under stirred conditions. Also, when the synthetic reaction is performed under milder conditions such as at a lower temperature and a lower pressure, the reaction is carried out in the presence of a catalyst which is active for the reaction of interest. A solid catalyst is often used.

Generally, the catalytic activities of these catalysts are correlated to certain physical and chemical characteristics of the catalyst, for example, the ability to adsorb components of the reaction system, surface area, pore structure, atomic arrangement of the surface, crystal lattice defects, and degree of acidity or basicity. This suggests that no solid catalyst being active for every reaction system can be prepared. Specifically, a specific catalyst having specific catalytic activities corresponds to a specific reaction system.

For example, a transition metal such as Ni, Pd, Pt, or the like is used for hydrogenation of an unsaturated hydrocarbon; a transition metal oxide such as $MoO_3$, $V_2O_5$ is used for selective oxidation of an organic compound; and $SiO_2$, $Al_2O_3$, zeolite, or the like as a solid catalyst is used for cracking of hydrocarbon or isomerization of a hydrocarbon.

In conventional methods for using a solid catalyst, one relies on the catalytic activities which are correlated to the above physical and chemical characteristics of the solid catalyst.
Hence there are limitations in the degree of activity. Also, there is no guarantee that a material exhibiting high catalytic activity for a desired synthetic reaction is always available.

There are cases where a desired reaction does not rapidly proceed, for example, at normal temperature even if a solid catalyst is utilized in a reaction system. In these cases, it is necessary to supply certain types of energy from outside the reaction system.

As the above energy, heat energy is conventionally used, specifically, to heat a reaction vessel externally and thereby to maintain a reaction system in a required high temperature condition.

The above reaction system includes the known reaction in which stable oxides of carbon such as $CO_2$, a carbonate ion ($CO_3^{2-}$), or the like are reduced to synthesize hydrocarbon or other useful organic compounds. The reaction system of this type has attracted remarkable attention as a method for absorbing $CO_2$ from an atmosphere to transform it to a useful compound by utilizing excess energy or natural energy though it has lately been apprehended that the increase in the concentration of $CO_2$ was a cause of the rise in the temperature of the earth.

The synthetic reaction in this reaction system produces CO according to the following formula (1) and produces a higher hydrocarbon according to the following formula (2), which are described in Catalyst Lecture Vol. 9, page 84, (edited by Catalyst Society; published by Kodan Co., Ltd.):

$$CO_2 + H_2 \rightarrow CO + H_2O \qquad (1)$$

$$nCO + (2n + 1)H_2 \rightarrow C_nH_{2n+2} + nH_2O \qquad (2)$$

Here, a temperature close to 1,000°C is required for the reaction of the formula (1) to proceed sufficiently. It is, therefore, necessary to heat a reaction vessel externally, resulting in large-scale installation.

Also, the reaction of the formula (2) is the so-called "Fisher-Tropsch reaction". This reaction proceeds by a method in which a catalyst is fed into a reaction vessel and CO and $H_2$ are introduced into the reaction vessel.

The reaction of the formula (2) is an exothermic reaction so that it is fundamentally unnecessary to supply energy required for reaction. However, it is necessary for the reaction system to be controlled at 150°C up to several hundred °C under normal pressure up to 10 MPa (10 atm) to sufficiently accelerate the reaction velocity even if a solid catalyst such as Co is fed.

However, even though the heat energy supplied externally to the reaction vessel is indispensable for activating the materials involved in the reaction, it is not always required for other materials. Even in this case, it is required to heat the entire reaction system uniformly by a conventional method for supplying heat energy.

In the conventional method for supplying heat energy, an excessively large amount of heat energy is supplied to the reaction system. There is the drawback of a large energy loss in relation to the supply of the heat energy to be required for the reaction system.

Also, the equipment for synthesis requires a large-scale installation and the system in which the heat energy is externally supplied indispensably involves high costs when measures are taken for dealing with problems of heat resistance and corrosion.

On the other hand, an electrolytic synthetic system is known as a peculiar synthetic system.

In this electrolytic synthetic system, a reaction vessel in which a pair of electrodes consisting of positive and negative poles are disposed is filled with a specific electrolyte. Voltage is applied between the electrodes so that an electrochemical reaction is carried out on the surface of the electrodes and a target compound is thereby synthesized.

In this reaction system, the energy required for the reaction is supplied directly to the materials in the reaction vessel in the form of electric energy. Therefore, it is unnecessary to heat the reaction vessel externally to supply heat energy to the reaction system even if the reaction system is an endothermic reaction system.

In this electrolytic synthetic system, the surface of the electrodes disposed in the electrolyte is the actual zone in which the synthetic reaction proceeds. In this system, therefore, the electrodes act as a kind of solid catalyst.

Incidentally, it is thought that the reactions of the above formulae (1) and (2) can be carried out by the electrolytic reaction system. In this case, because the energy to be supplied is electric energy, the reactions of the formulae (1) and (2) can be carried out at around normal temperature. In view of this, various electrodes as the solid catalyst have been investigated until now.

However, in almost all cases, only the reaction of the formula (1) proceeds to terminate the desired reaction or the products to be prepared are limited to very simple organic compounds such as methane, formic acid, and the like (see Chemistry and Chemical Industry, Vol. 43, page 2016-2017, 1990).

As one of such examples, a technique of producing methane by electrolytic synthesis is known (Goudal; French Patent Publication No. 2322115). According to this technique, an electrolytic aqueous solution is electrolyzed using an electrolytic cell provided with positive and negative electrodes. First, oxygen and hydrogen are generated at the positive and negative electrodes, respectively, and oxygen separated from the positive electrode is brought into contact with a carbonaceous fuel as a starting material, thereby causing oxidation to produce CO or $CO_2$. Subsequently, hydrogen is made to contact with the thus-produced CO or $CO_2$, thereby causing reduction to obtain $CH_4$. In this technique, however, electrode reaction is utilized merely to generate oxygen and hydrogen, and no synthesis is positively caused to take place on the surfaces of the electrodes. Consequently, no electric energy can be given to the starting material, and the final product obtained is limited to a simple organic compound, i.e. methane with the carbon number "1".

Specifically, it is difficult to carry out a C-C bonding reaction and a hydrogen abstracting reaction for forming a C=C bond from a C-C bond in the conventional electrolytic synthetic reaction system.

In addition, since the catalytic activities of the electrodes acting as the solid catalyst are also correlated to its physical and chemical characterization factors, it is required for heat energy to be supplied externally to the reaction system if the catalytic activity in the reaction system is insufficient.

Objects and Summary of the Invention

Accordingly, it is an object of the present invention to provide a process for preparing hydrocarbon compounds as indicated below, wherein such target compounds can be produced at normal temperature even if heat energy is not externally supplied to a reaction system when a synthetic reaction is carried out using a solid catalyst.

The above object can be attained in the present invention by providing a process for preparing hydrocarbon compounds comprising methane, ethane, propane, butane, pentane, hexane and heptane from $CO_2$ or $CO_3^{2-}$ and/or hydrocarbon compounds comprising ethane, propane, butane, pentane, hexane and heptane from $CH_4$ in an electrochemical reaction vessel containing positive and negative electrodes disposed in an aqueous electrolyte, said process comprising the steps of:

    applying voltage between said positive and negative electrodes to i nduce an electrolytic reaction of said electrolyte which causes a second reaction which is either a reaction in which oxygen generated from the positive electrode reacts with hydrogen or a metal on the surface of the negative electrode or a reaction in which hydrogen generated from the negative electrode reduces a metal oxide of the positive electrode or a combination of these second reactions,

    supplying to a zone on or close to the surface of the negative and/or positive electrode, where said second reaction takes place, at least one of $CO_2$, $CO_3^{2-}$ and $CH_4$ whereby said hydrocarbon compounds are formed.

In one embodiment of the invention,
    the electrolyte contains potassium hydroxide;
    the positive electrode includes nickel hydroxide;
    the negative electrode includes a hydrogen storage alloy; and
    oxygen generated at the positive electrode reacts on or close to the surface of the negative electrode with hydrogen stored in the hydrogen storage alloy of the negative electrode.

## Brief Description of the Drawings

FIG. 1 is a view showing a principal structure of a system B in an embodiment according to the present invention:

FIG. 2 is a view schematically showing the system B of the present invention arranged according to the Example 1:

FIG. 3 is a graph showing a gas chromatography result from Example 1:

FIG. 4 is a graph showing a gas chromatography result from Example 2:

FIG. 5 is a view showing a conventional system for performing electrolytic synthesis:

FIG. 6 is a graph showing a gas chromatography result for a synthesis carried out in the system illustrated in FIG. 5.

## Detailed Description of the Invention

The present invention will now be explained in detail with reference to the drawings.

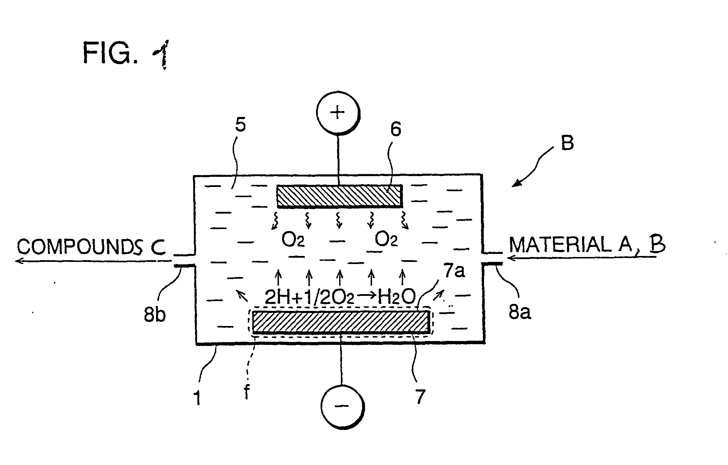

FIG. 1 is a view showing a principal structure of a system B in an embodiment according to the present invention.

This system B has a structure in which an electrolyte 5 described below is contained in a reaction vessel 1, a pair of electrodes consisting of a positive electrode 6 connected to a plus power source and a negative electrode 7 connected to a minus power source are disposed in the electrolyte 5, an introduction port 8a for introducing at least one of $CO_2$, $CO_3^{2-}$ and $CH_4$ (materials A, B) is provided at one side of the reaction vessel 1, and a discharge port 8b for discharging hydrocarbon compounds C (comprising methane, ethane, propane, butane, pentane, hexane and heptane) is provided at the other side of the reaction vessel 1.

In this system B, the above positive electrode 6 and/or negative electrode 7 act as a solid catalyst so-called in the present invention. The entire structure of the system B is similar to that of the system in which a gas component generated by one electrode is absorbed by the other electrode to restrain the rise of the internal pressure in a secondary battery when the secondary battery is excessively charged.

In this system B, voltage is applied between the electrodes 6 and 7 to carry out the electrolytic reaction of the electrolyte 5.

The above negative electrode 7 includes a metal such as Zn, Cd, Fe or the like or an oxide (hydroxide) of these metals, which is conventionally used as an active material for the negative electrode of an alkaline secondary battery, metal such as Co, Cu, Ni, Au, Ag, or the like or an oxide (hydroxide) of these metals, which is not an active material, and/or a hydrogen storage alloy or the like, and $O_2$ is primarily generated from the positive electrode 6. With the above structure, $O_2$ generated from the positive electrode 6 diffuses to the surface 7a of the negative electrode 7 to react with hydrogen or metal whereby $O_2$ is absorbed. As a result, a high energy sub-reaction zone f̲ is formed on or close to the surface 7a of the negative electrode 7.

Also, if a metal such as Ni, Ag, Mn, Co, Cu or the like or an oxide (hydroxide) of these metals is contained in the positive electrode 6 and $H_2$ is primarily generated from the negative electrode 7, a high energy sub-reaction zone f̲ is formed on or close to the surface 6a of the positive electrode 6 in the same manner as above.

Then, the synthesis of the product C comprising methane, ethane, propane, butane, pentane, hexane and heptane from $CO_2$ or $CO_3^{2-}$ and/or ethane, propane, butane, pentane, hexane and heptane from $CH_4$ is carried out by supplying at least one of $CO_2$, $CO_3^{2-}$ and $CH_4$ (materials A, B) in the sub-reaction zone f.

In one embodiment, the materials A and B may be introduced directly into the reaction vessel 1 in a fixed amount while the sub-reaction zone f̲ is stationarily formed. Also, if the materials A and B are soluble in the electrolyte 5, materials A and B are dissolved in the electrolyte in advance of forming the sub-reaction zone f̲. The latter is suitable because the desired main reaction proceeds smoothly and efficiently.

When the sub-reaction zone f̲ is formed using the above-described positive electrode 6 and the negative electrode 7, an alkaline electrolyte such as an aqueous KOH solution, aqueous NaOH solution, aqueous LiOH solution, or mixture of these are used since the component contained in the positive or negative electrode is an active material for alkaline secondary battery. There is a case in which it is suitable to use an acidic electrolyte depending on the kind of the reaction system. In such a case, an active material used for a lead battery is contained in the positive or negative electrode and an aqueous sulfuric acid solution is used as the electrolyte, whereby a similar sub-reaction zone can be formed.

In the system B, a porous separator may be arranged between the positive electrode 6 and the negative electrode 7 to avoid development of a short circuit across the two electrodes in the same manner as in the structure adopted for a secondary battery.

In addition, it is desirable that the vessel 1 be not filled with a liquid but be occupied with a gas to a certain degree so that a material required for forming the sub-reaction zone f̲, that is, a gas component generated from the positive electrode 6 or the negative electrode 7 can rapidly diffuse and move between the two electrodes and the sub-reaction can proceed at a high rate.

## Example 1

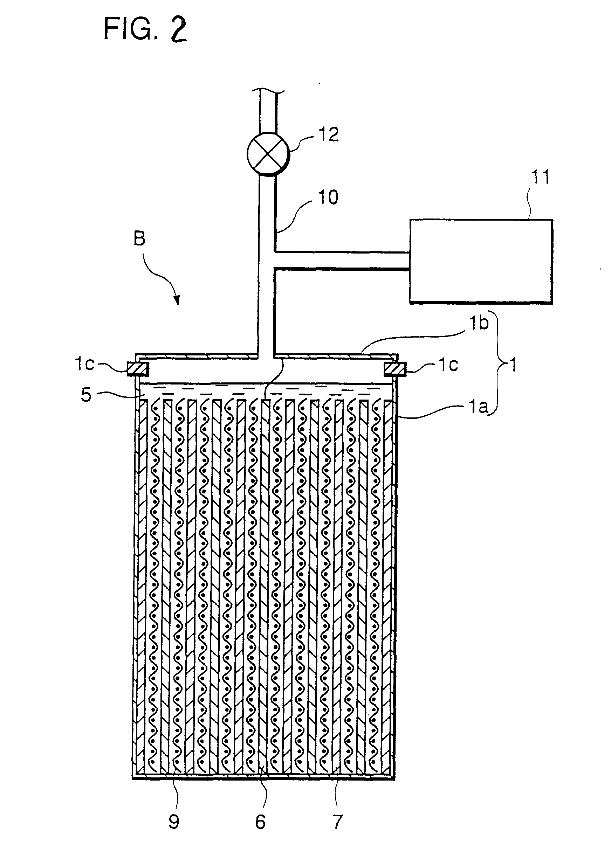

The system B shown in FIG. 2 was fabricated as follows:

1) Preparation of Positive electrode and Negative electrode

A nickel sponge with a porosity of 96% was filled with 4g of $Ni(OH)_2$ powder with an average diameter of approximately 10 μm and was then rolled to produce a positive electrode sheet of 70mm length, 40mm wide, 0.57mm thickness.

8g of a hydrogen storage alloy having the composition of $MmNi_{3.2}Co_{1.0}Al_{0.2}Mn_{0.4}$ (Mm is a misch metal containing 49% by weight of Ce and 25% by weight of La) together with a PTFE binding agent was applied to a metal sheet which comprises Ni plated stainless steel(numerical aperture: 40%, opening diameter: 1.5mm, thickness: 70 μm), which was then rolled to prepare a negative electrode sheet of 100mm length, 40mm wide, 0.35mm thickness.

2) Fabrication of system B

Next, as a separator 9, a polypropylene nonwoven fabric with a thickness of 0.15mm was sandwiched between the positive electrode 6 and negative electrode 7 and these were entirely wound up to a diameter of 13mm so that the negative electrode 7 was located externally.

The resulting construction was then inserted into a cylindrical can 1a with a bottom made of Ni plated stainless steel with an internal diameter of 140mm and a height of 500mm. In this case, the negative electrode 7 was inserted so that it contacted the internal wall of the cylindrical can 1a.

Then, a volume of the alkaline electrolyte 5 consisting of 5% by weight of $K_2CO_3$, 30% by weight of KOH, and 1.0% by weight of LiOH was charged into the cylindrical can 1a with a bottom. The upper opening of the cylindrical can was sealed with a lid 1b through an insulating material 1c. Here, the positive electrode 6 was electrically connected to the lid 1b.

A pressure sensor 11 and a closing valve 12 was connected to the lid 1b through thin pipe 10 having a diameter of 1 mm.

In this system B, the negative electrode 7 acted as a solid catalyst and the materials A and B were $CO_2$ ($CO_3^{2-}$) dissolved in the electrolyte 5.

3) Result of synthetic reaction

In the system B fabricated in this manner, a closing valve 12 was closed, the lid 1b was connected to a plus power source, the cylindrical can 1a with a bottom was connected to a minus power source, and 550 mA current was induced for 5 hours.

The electrolytic reaction of the electrolyte 5 was carried out by this energizing, whereby $O_2$ was primarily generated from the positive 6 and hydrogen was absorbed into the hydrogen storage alloy of the negative electrode 7. $O_2$ diffused and transferred to the negative electrode 7 and a sub-reaction took place on the electrode 7 whereby $O_2$ was combined with hydrogen in the alloy.

The pressure in the reaction vessel measured by a pressure sensor 11 during the energizing was maintained almost at 0.3 MPa (3 atm). Also, the temperature of the external wall of the cylindrical can 1a with a bottom was measured. The temperature was maintained almost at 35°C.

Namely, the above-described sub-reaction stationarily proceeded during the energizing. This sub-reaction was an exothermic reaction.

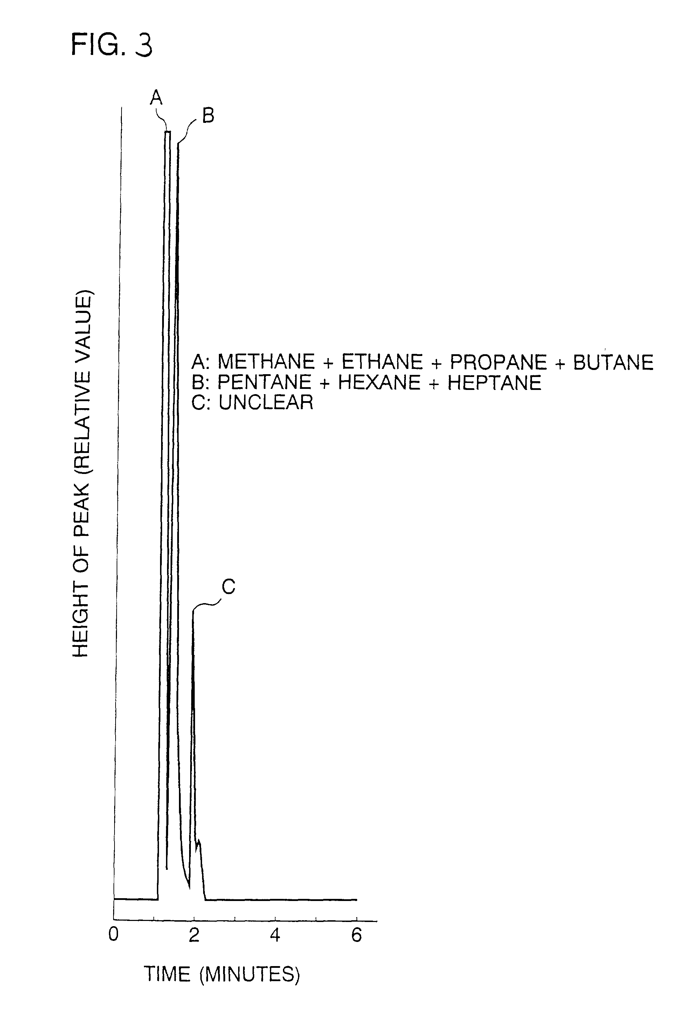

During the current induction, the closing valve 12 was opened intermittently to sample the gas in the vessel 1 from a thin pipe 10. The gas components were analyzed by means of gas chromatography. The results are shown in FIG. 3.

In FIG. 3, the peak A shows the presence of methane, ethane, propane, and butane and the peak B shows the presence of pentane, hexane, and heptane. Incidentally, which compounds peak C represents is not clear.

As is clear from FIG. 3, when the system B shown in FIG. 2, is operated, methane, ethane, propane, butane, pentane, hexane and heptane can be produced at 35°C, specifically close to room temperature.

Example 2

The system B was operated in the same manner as in Example 1 except that an alkaline electrolyte consisting of 30% by weight of KOH and 1.0% by weight of LiOH was used as the electrolyte and methane gas was introduced through the thin pipe 10 to seal up methane gas into the reaction system before energizing.

In this case, the pressure in the reaction vessel 1 during the energizing was maintained almost at 0.3 MPa (3 atms). Also, the temperature of the external wall of the cylindrical can 1a was maintained almost at 35°C.

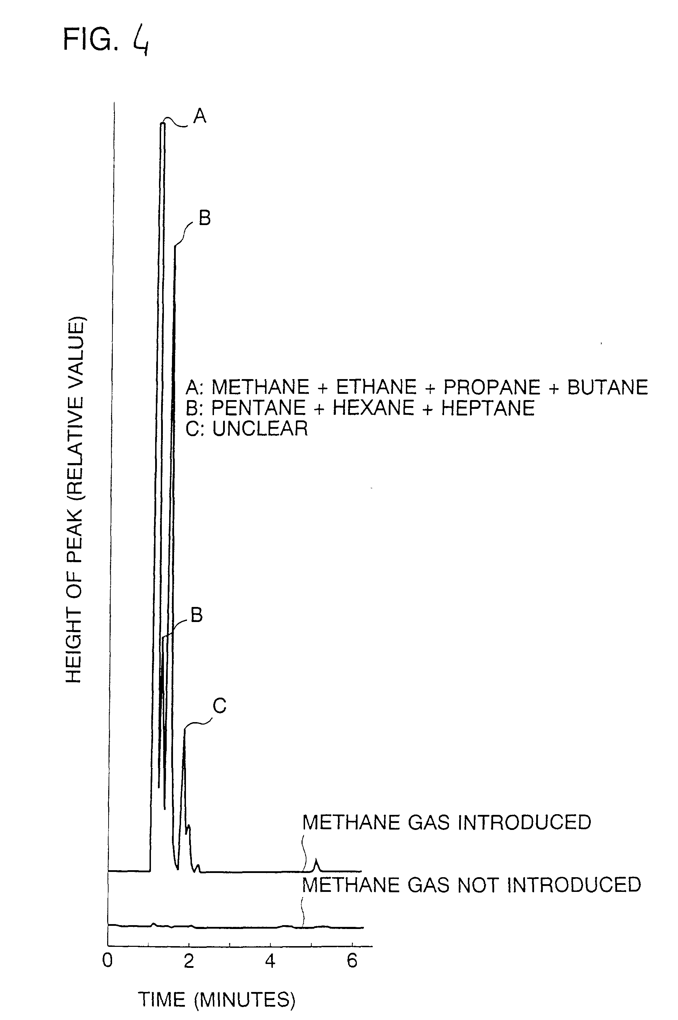

Gas components in the reaction vessel 1 were analyzed by means of gas chromatography in the same manner as in Example 1. The results are shown in FIG. 4. In FIG. 4, the results of gas chromatography in the case where methane was not sealed up into the reaction system also was shown for comparison.

As is clear from FIG. 4, with the system B of the present invention, ethane, propane, butane, pentane, hexane, and heptane were synthesized using methane gas as a starting material at 35°C, specifically close to room temperature.

Comparative Example

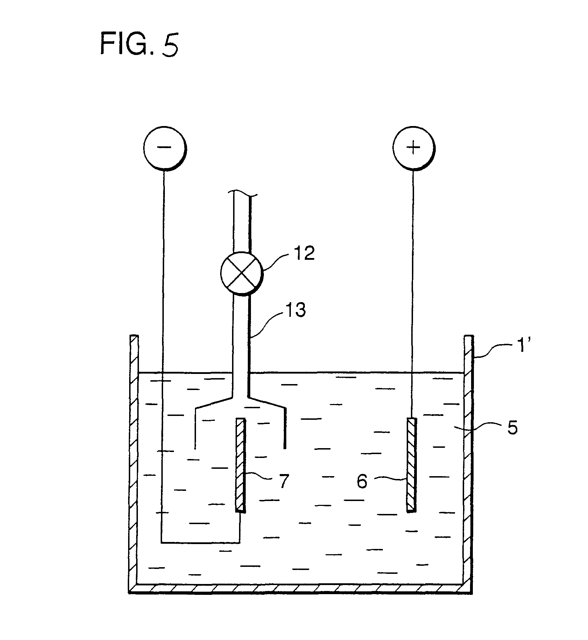

A conventional electrolytic synthesis was tried for comparison. Specifically, as shown in FIG. 5, an electrolytic vessel l' was filled with an electrolyte 5 used in Example 1. A positive electrode 6 and a negative electrode 7 used in Example 1 were disposed opposite each other.

A cover 13 provided with a closing valve 12 is disposed so that it entirely covers the negative electrode 7. In this condition, the two electrodes were energized under the same condition as in Example 1.

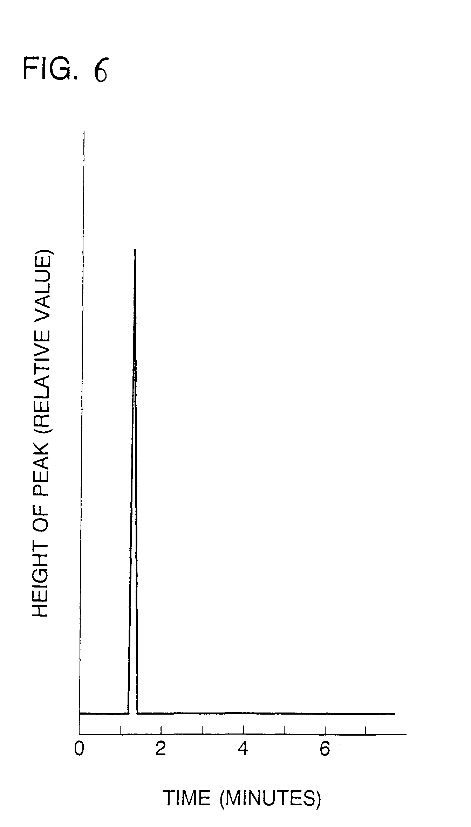

The closing valve 12 was opened, generated gas was sampled from the cover 13, and it was analyzed by means of gas chromatography. The results are shown in FIG. 6.

One peak was observed. This peak shows the presence of methane.

As is clear from the above explanations, in the present invention, even if the reaction system is not high in temperature by externally heating the reaction system, a target compound can be obtained at around normal temperature.

This effect is based on carrying out a sub-reaction as described above releasing energy on the surface of a solid catalyst and carrying out a main reaction as described above producing targeted compounds in the sub-reaction field.

**Claims**

1. A process for preparing hydrocarbon compounds comprising methane, ethane, propane, butane, pentane, hexane and heptane from $CO_2$ or $CO_3^{2-}$ and/or hydrocarbon compounds comprising ethane, propane, butane, pentane, hexane and heptane from $CH_4$ in an electrochemical reaction vessel containing positive and negative electrodes disposed in an aqueous electrolyte, said process comprising the steps of:

    applying voltage between said positive and negative electrodes to induce an electrolytic reaction of said electrolyte which causes a second reaction which is either a reaction in which oxygen generated from the positive electrode reacts with hydrogen or a metal on the surface of the negative electrode or a reaction in which hydrogen generated from the negative electrode reduces a metal oxide of the positive electrode or a combination of these second reactions,

    supplying to a zone on or close to the surface of the negative and/or positive electrode, where said second reaction takes place, at least one of $CO_2$, $CO_3^{2-}$ and $CH_4$ whereby said hydrocarbon compounds are formed.

2. A process according to Claim 1, wherein the negative electrode includes at least one selected from the group consisting of Zn, Fe, Cd, Co, Cu, Pb, Ni, Pt, Pd, Au, Ag, and oxides or hydroxides of these and a hydrogen storage alloy or its hydride, or both.

3. A process according to Claim 1, wherein the positive electrode includes at least one selected from the group consisting of oxides or hydroxides of a metal such as Ni, Mn, Co, Cu, and Ag; or one selected from the group consisting of Ni, Pt, Pd, and Cu; or both.

4. A process according to Claim 1, wherein the electrolyte is at least one of an electrolyte selected from the group consisting of an aqueous potassium hydroxide solution, aqueous sodium hydroxide solution, and aqueous lithium hydroxide solution; or an aqueous sulfuric acid.

5. A process according to Claim 1, wherein a porous separator is disposed between the positive electrode and the negative electrode.

6. A process according to Claim 1, wherein

    the electrolyte contains potassium hydroxide;

    the positive electrode includes nickel hydroxide;

    the negative electrode includes a hydrogen storage alloy;

    and

    oxygen generated at the positive electrode reacts on or close to the surface of the negative electrode with hydrogen stored in the hydrogen storage alloy of the negative electrode.

**Patentansprüche**

1. Verfahren zur Herstellung von Kohlenwasserstoffverbindungen, die Methan, Ethan, Propan, Butan, Pentan, Hexan und Heptan umfassen, aus $CO_2$ oder $CO_3^{2-}$ und/oder Kohlenwasserstoffverbindungen, die Ethan, Propan, Butan, Pentan, Hexan und Heptan umfassen, aus $CH_4$ in einem elektrochemischen Reaktionsgefäß, das positive und negative Elektroden enthält, die in einem wässrigen Elektrolyten angeordnet sind, wobei das Verfahren folgende Schritte umfaßt:

    - Anlegen einer Spannung zwischen den positiven und negativen Elektroden, um eine elektrolytische Reaktion des Elektrolyten herbeizuführen, die eine zweite Reaktion hervorruft, die entweder eine Reaktion ist, bei der von der positiven Elektrode erzeugter Sauerstoff mit Wasserstoff oder einem Metall auf der Oberfläche der negativen Elektrode reagiert, oder eine Reaktion, bei der von der negativen Elektrode erzeugter Wasserstoff ein Metalloxid der positiven Elektrode reduziert, oder eine Kombination dieser zweiten Reaktionen,

    Zuführen von mindestens einem von $CO_2$,

$CO_3^{2-}$ und $CH_4$ an eine Zone auf oder nahe der Oberfläche der negativen und/oder positiven Elektrode, an der die zweite Reaktion stattfindet, wodurch die Kohlenwasserstoffverbindungen gebildet werden.

2. Verfahren nach Anspruch 1, bei dem die negative Elektrode mindestens eines einschließt, das gewählt ist aus der Gruppe bestehend aus Zn, Fe, Cd, Co, Cu, Pb, Ni, Pt, Pd, Au, Ag und Oxiden oder Hydroxiden davon und einer Wasserstoffspeicherlegierung oder ihrem Hydrid, oder beidem.

3. Verfahren nach Anspruch 1, bei dem die positive Elektrode mindestens eines einschließt, das gewählt ist aus der Gruppe bestehend aus Oxiden oder Hydroxiden eines Metalls wie z.B. Ni, Mn, Co, Cu und Ag; oder eines gewählt aus der Gruppe bestehend aus Ni, Pt, Pd und Cu; oder beides.

4. Verfahren nach Anspruch 1, bei dem der Elektrolyt mindestens einer eines Elektrolyten ist, der gewählt ist aus der Gruppe bestehend aus einer wässrigen Kaliumhydroxidlösung, wässrigen Natriumhydroxidlösung und wässrigen Lithiumhydroxidlösung; oder eine wässrige Schwefelsäure.

5. Verfahren nach Anspruch 1, bei dem ein poröser Separator zwischen der positiven Elektrode und der negativen Elektrode angeordnet ist.

6. Verfahren nach Anspruch 1, bei dem
der Elektrolyt Kaliumhydroxid enthält;
die positive Elektrode Nickelhydroxid einschließt;
die negative Elektrode eine Wasserstoffspeicherlegierung einschließt; und
an der positiven Elektrode erzeugter Sauerstoff auf oder nahe der Oberfläche der negativen Elektrode mit in der Wasserstoffspeicherlegierung der negativen Elektrode gespeichertem Wasserstoff reagiert.

**Revendications**

1. Procédé pour préparer des composés hydrocarbonés comprenant le méthane, l'éthane, le propane, le butane, le pentane, l'hexane et l'heptane à partir de $CO_2$ ou $CO_3^{2-}$ et/ou des composés hydrocarbonés comprenant l'éthane, le propane, le butane, le pentane, l'hexane et l'heptane à partir de $CH_4$ dans un récipient réactionnel électrochimique contenant des électrodes positive et négative disposées dans un électrolyte aqueux, ledit procédé comprenant les étapes de :

appliquer une tension entre lesdites électrodes positive et négative pour induire une réaction électrolytique dudit électrolyte qui provoque une seconde réaction qui est soit une réaction dans laquelle l'oxygène engendré à partir de l'électrode positive réagit avec l'hydrogène ou un métal sur la surface de l'électrode négative, ou une réaction dans laquelle l'hydrogène engendré à partir de l'électrode négative réduit un oxyde métallique de l'électrode positive, ou une combinaison de ces secondes réactions,
fournir à une zone sur ou proche de la surface de l'électrode négative et/ou positive, où ladite seconde réaction a lieu, au moins l'un de $CO_2$, $CO_3^{2-}$ et $CH_4$, grâce à quoi lesdits composés hydrocarbonés sont formés.

2. Procédé selon la revendication 1, dans lequel l'électrode négative inclut au moins un choisi à partir du groupe constitué de Zn, Fe, Cd, Co, Cu, Pb, Ni, Pt, Pd, Au, Ag, et d'oxydes ou hydroxydes de ceux-ci et un alliage de stockage d'hydrogène ou son hydrure, ou les deux.

3. Procédé selon la revendication 1, dans lequel l'électrode positive inclut au moins un choisi à partir du groupe constitué d'oxydes ou d'hydroxydes d'un métal tel que Ni, Mn, Co, Cu et Ag ; ou un choisi à partir du groupe constitué de Ni, Pt, Pd et Cu ; ou les deux.

4. Procédé selon la revendication 1, dans lequel l'électrolyte est au moins un d'un électrolyte choisi à partir du groupe constitué d'une solution d'hydroxyde de potassium aqueuse, d'une solution d'hydroxyde de sodium aqueuse et d'une solution d'hydroxyde de lithium aqueuse ; ou un acide sulfurique aqueux.

5. Procédé selon la revendication 1, dans lequel un séparateur poreux est disposé entre l'électrode positive et l'électrode négative.

6. Procédé selon la revendication 1, dans lequel
l'électrolyte contient de l'hydroxyde de potassium ;
l'électrode positive inclut de l'hydroxyde de nickel ;
l'électrode négative inclut un alliage à stockage hydrogène ; et
l'oxygène engendré au niveau de l'électrode positive réagit sur ou proche de la surface de l'électrode négative avec l'hydrogène stocké dans l'alliage de stockage d'hydrogène de l'électrode négative.

FIG. 1

FIG. 2

## FIG. 3

A: METHANE + ETHANE + PROPANE + BUTANE
B: PENTANE + HEXANE + HEPTANE
C: UNCLEAR

HEIGHT OF PEAK (RELATIVE VALUE)

TIME (MINUTES)

FIG. 4

A: METHANE + ETHANE + PROPANE + BUTANE
B: PENTANE + HEXANE + HEPTANE
C: UNCLEAR

METHANE GAS INTRODUCED

METHANE GAS NOT INTRODUCED

HEIGHT OF PEAK (RELATIVE VALUE)

TIME (MINUTES)

FIG. 5

## FIG. 6